(19) **Europäisches Patentamt European Patent Office Office européen des brevets**

(11) **EP 2 249 870 B1**

(12) **FASCICULE DE BREVET EUROPEEN**

(45) Date de publication et mention
de la délivrance du brevet:
**18.03.2015 Bulletin 2015/12**

(21) Numéro de dépôt: **09718722.3**

(22) Date de dépôt: **20.02.2009**

(51) Int Cl.:
*A61K 47/26* (2006.01)   *A23L 1/236* (2006.01)
*A23G 1/40* (2006.01)   *A23G 3/42* (2006.01)
*A23G 4/10* (2006.01)   *A23L 1/307* (2006.01)

(86) Numéro de dépôt international:
**PCT/FR2009/050276**

(87) Numéro de publication internationale:
**WO 2009/112740 (17.09.2009 Gazette 2009/38)**

(54) **POUDRE DE MALTITOL CRISTALLISÉ DE GROSSE GRANULOMETRIE, SON PROCEDE DE FABRICATION ET SES APPLICATIONS, NOTAMMENT DANS LE CHOCOLAT**

KRISTALLISIERTES MALTITOLPULVER MIT GROSSER PARTIKELGRÖSSE, HERSTELLUNGSVERFAHREN UND VERWENDUNG DAFÜR, IM BESONDEREN IN DER SCHOKOLADENPRODUKTION

LARGE PARTICLE SIZE CRYSTALLISED MALTITOL POWDER, METHOD FOR PREPARING SAME AND APPLICATIONS THEREOF, PARTICULARLY IN CHOCOLATE PRODUCTION

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **22.02.2008 FR 0851168**

(43) Date de publication de la demande:
**17.11.2010 Bulletin 2010/46**

(73) Titulaire: **Roquette Freres
62136 Lestrem (FR)**

(72) Inventeurs:
• **BARATA, Manuel
F-62920 Gonnehem (FR)**
• **LE BOT, Yves
F-59139 Wattignies (FR)**
• **MULLER née OSTERMANN, Elsa
F-62920 Gonnehem (FR)**
• **RIBADEAU-DUMAS, Guillaume
F-59237 Verlinghem (FR)**

(74) Mandataire: **Cabinet Plasseraud
52, rue de la Victoire
75440 Paris Cedex 09 (FR)**

(56) Documents cités:
**EP-A- 0 735 042      EP-A- 1 207 164
WO-A-2004/067595      US-A- 1 861 248**

**Description**

La présente invention est relative à un procédé de préparation d'une poudre de maltitol cristallisé de richesse élevée en maltitol et de grosse granulométrie, pratiquement dépourvue de fines particules. Cette poudre de maltitol cristallisé est également caractérisée par son excellente aptitude à l'écoulement et sa densité.

Au sens de l'invention, on entend par « poudre de maltitol cristallisé », le produit de la cristallisation classique d'une solution aqueuse de maltitol.

Au sens de l'invention, on entend par « richesse élevée en maltitol » une richesse en maltitol supérieure à 99,5 % en poids, de préférence supérieure à 99,7 % en poids, et plus préférentiellement encore supérieure à 99,8 % en poids.

Le 4-O-alpha-D-glucopyranosyl-D-glucitol, appelé communément maltitol, est un polyol obtenu industriellement par hydrogénation du maltose. Il présente un grand intérêt en raison du fait qu'il est plus stable chimiquement, moins calorique, et qu'il présente un index glycémique plus bas que le saccharose, tout en possédant avantageusement des propriétés organoleptiques très voisines de celles de ce sucre. De plus, le maltitol possède la particularité de n'être pas cariogène, ce qui lui ouvre et lui a déjà ouvert de multiples applications dans l'industrie, notamment dans les industries pharmaceutiques et alimentaires, notamment dans les domaines du chewing gum, des édulcorants de table et du chocolat.

Dans le domaine du chocolat, par exemple, on distingue trois types de chocolat : le chocolat noir, le chocolat au lait et le chocolat blanc.

D'une façon générale, un chocolat noir traditionnel peut être défini comme un produit obtenu à partir de liqueur de cacao (contenant environ 54 % de matières grasses), de saccharose et de beurre de cacao. Il est fréquent d'utiliser également un émulsifiant comme la lécithine ainsi que parfois de la poudre de cacao, et éventuellement un arôme.

Le chocolat au lait comporte en plus de la matière sèche de lait, et le chocolat blanc en comprend également mais est exempt de poudre de cacao sec dégraissé.

D'un point de vue physique, un chocolat peut être assimilé à une dispersion presque anhydre de très fines particules non grasses (saccharose, lactose, protéines, minéraux) dans une phase grasse solidifiée, constituée essentiellement de triglycérides.

Ces derniers sont issus uniquement du cacao dans le cas d'un chocolat noir, mais proviennent également du lait dans le cas du chocolat au lait ou blanc.

Un procédé classique de fabrication de chocolat comprend les étapes essentielles successives suivantes :

- pétrissage,
- raffinage,
- éventuellement conchage à sec
- conchage liquide,
- tempérage,
- moulage,
- refroidissement,
- conditionnement.

Le pétrissage vise à obtenir une pâte homogène à partir de sucre, de pâte de cacao et éventuellement de beurre de cacao et de poudre de lait.

L'opération s'effectue dans un pétrin mécanique. La pâte obtenue doit présenter une texture particulière, adaptée à l'opération ultérieure de raffinage.

Il est possible d'ajuster celle-ci par le choix de la granulométrie du sucre et également par la teneur en matières grasses.

Le raffinage consiste à laminer la pâte obtenue en sortie de pétrissage entre des cylindres en acier, de façon à réduire la taille des particules à moins de 25 ou 30 microns environ.

Cette opération transforme la pâte initiale en poudre fine, hygroscopique et susceptible de piéger les odeurs ambiantes.

A ce stade, il est donc souhaitable que le conchage intervienne aussi rapidement que possible.

Le conchage est essentiel pour modifier l'arôme et améliorer les caractéristiques rhéologiques du chocolat.

Cette opération peut avoir lieu, en une seule étape (conchage liquide) ou en deux étapes (conchage à sec puis liquide) et durer de quelques heures à plusieurs jours.

La poudre raffinée est malaxée à chaud, vers 75-80°C dans le cas d'un chocolat noir, et vers 65°C pour les chocolats blanc et au lait. Le conchage à sec consiste à réaliser ce malaxage à chaud en l'absence de fortes teneurs en matières grasses. Il permet de réduire les temps de conchage.

Au cours de cette opération, l'arôme du chocolat se développe. Grâce à l'élévation de température et à l'aération de la masse mises en oeuvre, des composés indésirables comme les aldéhydes et acides gras courts s'échappent de la masse par volatilisation, alors que d'autres composés aromatiques sont formés.

De plus, la rhéologie du produit change : la poudre obtenue à l'issue du raffinage évolue vers l'état pâteux. Les particules insolubles (de sucre, de cacao, de lait) se dissocient par friction et départ d'eau et s'arrondissent pour communiquer à la pâte une plus grande fluidité avec un seuil d'écoulement plus faible.

Pour améliorer encore ces caractéristiques, de la lécithine est généralement ajoutée au chocolat quelques heures avant la fin du conchage.

Celle-ci enrobe les particules sucrées et émulsifie les traces d'eau résiduelle pour donner au chocolat de bonnes propriétés d'écoulement qui sont essentielles pour l'étape ultérieure de coulage.

Le tempérage du chocolat est destiné à permettre une cristallisation du beurre de cacao sous forme stable.

Pour cela, la pâte chocolatée est amenée à une température voisine de 25 à 27°C, parfois légèrement inférieure, de façon à créer des amorces cristallines de toute nature, puis à une température légèrement plus élevée lors du coulage, afin de faire fondre les formes cristallines instables.

Le coulage est une opération de mise en forme du chocolat, par exemple en tablettes ou en figurines. Il peut s'agir de chocolats pleins ou fourrés.

Le saccharose est, depuis les débuts de l'industrie du chocolat, la charge sucrante de référence. Ses propriétés sensorielles et technologiques le rendent particulièrement adapté à ce type de produit de confiserie.

En revanche, ses propriétés nutritionnelles peuvent donner lieu à des critiques. En effet, le saccharose possède une valeur calorique de 4 kcal/g, ce qui confère au chocolat dont le saccharose est le constituant essentiel, une valeur calorique non négligeable.

En outre, il est connu que le sucre est tout à fait contre-indiqué pour les diabétiques car le glucose qui le constitue pour partie est rapidement assimilable par l'organisme, ce qui peut générer de graves hyperglycémies chez ces malades.

Enfin, le saccharose est un substrat fermentescible par les bactéries commensales de la bouche, qui le transforment en acides corrosifs à l'origine des caries dentaires.

Pour pallier ces inconvénients, il a été imaginé de substituer le saccharose par les polyols dans le chocolat.

Ces polyols peuvent être notamment des monosaccharides hydrogénés tels que le sorbitol, le mannitol, le xylitol, l'érythritol ou des disaccharides hydrogénés tels que le maltitol, le lactitol, l'isomaltulose hydrogéné (mélange équimoléculaire de glucopyranosyl-1-6 sorbitol et de glucopyranosyl 1-1 mannitol).

A l'état pur, ces polyols n'ont pas de pouvoir réducteur et ne sont pas fermentés par la flore buccale en acides. Ils permettent donc la fabrication de chocolats non cariogènes dans la mesure où les autres ingrédients de la formulation n'apportent pas de sucres fermentescibles. Dans le cas de chocolats au lait et de chocolats blancs, le lait peut être remplacé par des ingrédients laitiers délactosés afin d'assurer au mieux cette hypocariogénicité.

Les polyols sont lentement métabolisés et n'entraînent pas après leur consommation une élévation brutale du taux de glucose dans le sang. En conséquence, ils sont souvent recommandés dans l'alimentation des diabétiques.

De plus, leur valeur calorique est estimée en moyenne à 2,4 kcal/g (10,0 KJ/g) soit environ 60 % de celle du sucre.

Cependant, en ce qui concerne l'allègement calorique, on ne peut que constater que celui-ci reste encore limité pour les chocolats aux polyols commercialisés actuellement, et ce pour la simple raison qu'à la valeur calorique de charge édulcorante s'ajoute celle, beaucoup plus importante, des matières grasses qui constituent un autre ingrédient essentiel du chocolat.

Ces matières grasses proviennent généralement du cacao et/ou du lait.

Leur valeur calorique s'élève en effet à 9 kcal/g. De plus, elles sont essentiellement sous forme saturée. Elles ne sont donc pas particulièrement recommandées par les nutritionnistes et vont à l'encontre du souci actuel des consommateurs, qui est de limiter l'apport excessif de calories par l'alimentation.

Pour répondre à ce souci, dans le cas du chocolat, il convient donc de remplacer le saccharose par un substitut faiblement calorique, les polyols étant notamment à ce titre tout à fait appropriés, mais également de réduire la quantité de matières grasses.

Or, il existe des impératifs technologiques de fabrication dont notamment les caractéristiques rhéologiques nécessaires pour procéder dans de bonnes conditions aux opérations de raffinage, de conchage et de coulage, qui a priori s'opposent à une réduction significative de la teneur en matières grasses dans les chocolats aux polyols.

Une solution technique a été apportée à ces difficultés par la société Demanderesse dans son brevet EP 512.910, solution reposant sur la mise au point d'un chocolat hypocalorique qui, bien que présentant une teneur en matières grasses très réduite, puisque inférieure à 32 % en poids, présentait des propriétés technologiques et organoleptiques comparables à celles du chocolat au saccharose traditionnel : utiliser pour la constitution de la charge édulcorante des produits sélectionnés dans le groupe constitué par le maltitol cristallisé de haute pureté, le lactitol, l'isomaltulose hydrogéné, les polymères de saccharides hypocaloriques, ou leurs mélanges.

Il était cependant entendu dans la demande de brevet EP 512.910, par « maltitol cristallisé de haute pureté » :

- du maltitol cristallisé présentant une teneur en maltitol exprimée en poids sec/sec d'au moins 92 %, de préférence d'au moins 95 %, et plus préférentiellement encore d'au moins 97 %,

- tel que celui obtenu selon le procédé de fabrication décrit dans le brevet européen EP 189.704 dont la société Demanderesse est la propriétaire (le protocole de cristallisation dans l'eau par refroidissement est décrit dans l'exemple de ladite demande de brevet).

Ce « maltitol cristallisé de haute pureté » obtenu par la société Demanderesse présente classiquement, pour un diamètre moyen arithmétique compris entre 180 et 230 $\mu$m, une répartition granulométrique en volume, caractérisée par :

- plus de 20 % de particules présentant une taille inférieure à 200 $\mu$m,
- plus de 7 % de particules de taille inférieure à 100 $\mu$m,
- plus de 2 % de particules de taille inférieure à 40 $\mu$m (les mesures sont déterminées par granulométrie laser, comme il sera explicité ci-après).

Ce produit est par ailleurs commercialisé par la société Demanderesse sous le nom de marque MALTISORB® P200.

Si ce « maltitol cristallisé de haute pureté » convient tout à fait à l'application chocolat, notamment pour la fabrication d'un chocolat hypocalorique, la société Demanderesse a constaté que la présence des fines particules de maltitol cristallisé de moins de 200 $\mu$m, plus particulièrement de moins de 100 $\mu$m, et plus particulièrement encore de moins de 40 $\mu$m pouvait s'avérer préjudiciable aux conditions de fabrication d'un chocolat à très faible teneur en matières grasses, notamment pendant la phase de malaxage, avant le passage à l'étape de raffinage.

Dans le domaine des édulcorants de table, la société Demanderesse a également proposé dans son brevet EP 1.245.582 des édulcorants de table en poudre enrichis en fibres, pouvant notamment contenir des polyols.

Les édulcorants de table s'entendent habituellement de compositions de remplacement de sucres traditionnels (saccharose) sous forme poudre qui présentent un pouvoir sucrant comparable ou supérieur à celui du saccharose, pour une valeur calorique du même ordre de grandeur (de l'ordre de 4 Kcal/g) voire plus faible.

Du fait de leur pouvoir sucrant en général supérieur, les quantités d'édulcorants de table nécessaires pour sucrer les aliments ou les boissons sont ainsi plus faibles que celles requises avec le saccharose, ce qui réduit d'autant la charge calorique pour un même pouvoir sucrant.

Le pouvoir édulcorant est par exemple apporté par des agents édulcorants intenses préparés par synthèse chimique de type saccharine, aspartame, acésulfame K, cyclamate, stévioside, sucralose, néotame ou alitame.

Ces édulcorants de table renferment également avec l'agent édulcorant, des agents de charge, habituellement choisis parmi les polyols, tels par exemple le sorbitol, le xylitol, le mannitol, le lactitol, le maltitol, l'érythritol et l'isomalt pris seuls ou en mélange, ou encore des polysaccharides ou des oligosaccharides de type dextrines, maltodextrines, polydextrose ou fructooligosaccharides.

Les édulcorants de table sont utilisés de manière intensive dans les industries des aliments et de la restauration, notamment sous forme poudre, pour leur apport de goûts sucrés sans apport calorique élevé.

De tels édulcorants de table sont ainsi largement répandus dans les aliments dits diététiques ou « light », destinés aux agents amincissants ou autres agents à valeur calorique contrôlée.

Dans son brevet EP 1.245.582, la société Demanderesse propose des édulcorants de table enrichis en fibres, caractérisés en ce qu'ils comprennent de 3 à 99 %, et de préférence de 10 à 95% en poids de maltodextrines branchées présentant entre 15 et 35 % de liaisons glucosidiques 1-6, une teneur en sucres réducteurs inférieure à 20 %, un indice de polymolécularité inférieur à 5 et une masse moléculaire moyenne en nombre Mn au plus égale à 4500 g/mole, et qu'ils sont stables en conditions acides.

Par « maltodextrines branchées », il faut entendre des maltodextrines décrites dans la demande de brevet EP 1.006.128 dont la société Demanderesse est également titulaire.

Ces maltodextrines branchées présentent un caractère d'indigestibilité qui a pour conséquence de diminuer leur pouvoir calorique, en empêchant leur assimilation au niveau de l'intestin grêle. Elles constituent donc une source de fibres indigestibles.

Dans son brevet EP 1.245.582, la société Demanderesse a trouvé que l'incorporation desdites maltodextrines branchées permet avantageusement une substitution partielle ou totale des agents de charge dans les édulcorants de table enrichis en fibres de manière à en réduire la cariogénicité, et en constituant un apport de fibres indigestibles dans une application à laquelle ne pouvaient prétendre les fructooligosaccharides ou les polydextroses classiquement utilisés par ailleurs.

Ainsi, en substituant par exemple dans une composition édulcorante calorique tout ou partie des maltodextrines par les maltodextrines branchées, on peut obtenir une composition allégée jusqu'à 50 % de sa valeur calorique initiale, présentant des qualités organoleptiques satisfaisantes.

Toutes les compositions décrites dans la demande de brevet EP 1.006.128 sont appropriées à la préparation d'édulcorants de table selon l'invention.

En plus des maltodextrines branchées et desdits édulcorants intenses, ces édulcorants de tables enrichis en fibres proposés par la société Demanderesse renferment un complément de polyols.

Ces polyols sont avantageusement choisis dans le groupe constitué par le saccharose, le sorbitol, le xylitol, le mannitol, le maltitol, l'isomalt, le lactitol et l'érythritol, pris seul ou en combinaison.

Selon une variante avantageuse, lesdits agents édulcorants de table enrichis en fibres conformes à l'invention comprennent de 3 à 50 % en poids desdites maltodextrines branchées, le complément en poids à 100 % étant un sucre ou un polyol choisi dans le groupe constitué par le saccharose, le fructose, le dextrose, le maltose, les sirops de glucose déshydratés, le maltitol, le lactitol, le mannitol, le xylitol, le sorbitol, l'érythritol, l'isomalt, le thréitol et l'iditol pris seuls ou en combinaison.

Dans le cas particulier du maltitol, la société Demanderesse a également constaté que la présence de fines particules de maltitol cristallisé, de moins de 200 $\mu$m, plus particulièrement de moins de 100 $\mu$m et plus particulièrement encore de moins de 40 $\mu$m pouvait s'avérer préjudiciable aux conditions de fabrication desdits édulcorants de table.

On constate en effet que plus la concentration en maltodextrines branchées sera élevée, moins la poudre de maltitol cristallisé, si elle est riche en particules de fine granulométrie, s'agglomérera facilement.

Par ailleurs, la société Demanderesse a également constaté que pour cette application édulcorant de table, l'aptitude à l'écoulement et la densité de la poudre de maltitol sont des paramètres importants.

De tout ce qui précède, il résulte qu'il demeure un besoin non satisfait de disposer d'une poudre de maltitol cristallisé qui présente d'une part une granulométrie élevée pratiquement dépourvue de fines particules, ce qui la destine plus particulièrement aux domaines du chocolat (notamment dans le chocolat à faible teneur en matières grasses), et qui présente d'autre part une bonne aptitude à l'écoulement et une densité élevée, ce qui la destine plus particulièrement dans le domaine des édulcorants de table.

Il est du mérite de la société Demanderesse d'avoir réussi, après avoir mené une recherche approfondie sur le sujet, à développer un procédé de préparation d'une poudre de maltitol cristallisé ne présentant pas les défauts relevés pour les poudres de maltitol connues.

La poudre de maltitol cristallisé présente une richesse en maltitol supérieure à 99,5 % en poids, de préférence supérieure ou égale à 99,7 % en poids, plus préférentiellement supérieure à 99,8 % en poids, caractérisée par :

- une répartition granulométrique en volume, déterminée par granulométrie laser, présentant :

  - moins de 20 %, de préférence moins de 15 %, plus préférentiellement moins de 10 % et plus préférentiellement encore moins de 5 % de particules de taille inférieure à 200 $\mu$m,
  - moins de 6 % de particules de taille inférieure à 100 $\mu$m,
  - moins de 2 % de particules de taille inférieure à 40 $\mu$m, et

- une valeur d'écoulement inférieure ou égale à 10 secondes, de préférence inférieure ou égale à 5 secondes,
- une densité aérée supérieure à 0,85 g/ml, de préférence comprise entre 0,88 et 1,00 g/ml, une densité tassée supérieure à 0,97 g/ml, de préférence comprise entre 0,98 et 1,05 g/ml et une compressibilité inférieure à 17 %, de préférence inférieure à 10 %, et plus préférentiellement encore inférieure à 5 %.

Les valeurs de répartition granulométrique sont déterminées sur un granulomètre à diffraction LASER type LS 230 de la société BECKMAN-COULTER, équipé de son module de dispersion poudre (voie sèche), en suivant le manuel technique et les spécifications du constructeur.

Les conditions opératoires de vitesse de vis sous trémie et d'intensité de vibration de la goulotte de dispersion sont déterminées de manière à ce que la concentration optique soit comprise entre 4 % et 12 %, idéalement 8 %.

La gamme de mesure du granulomètre à diffraction LASER type LS 230 est de 0,04 $\mu$m à 2.000 $\mu$m. Les résultats sont calculés en % volumique, et exprimés en $\mu$m.

La courbe de distribution granulométrique permet également de déterminer la valeur du diamètre moyen volumique (moyenne arithmétique) D4,3.

La poudre de maltitol cristallisé obtenue par le procédé de l'invention est surtout caractérisée par la faible teneur en particules de petite taille.

Plus particulièrement, la très faible teneur de particules de taille inférieure à 100 $\mu$m confère à la poudre de maltitol cristallisé obtenue par le procédé conforme à l'invention des propriétés d'écoulement remarquables.

La poudre de maltitol cristallisé obtenue par le procédé conforme à l'invention est par ailleurs caractérisée par :

- son aptitude à l'écoulement,
- sa densité (aérée et tassée) et sa compressibilité.

Les valeurs d'écoulement sont déterminées selon la méthode de mesure préconisée par la Pharmacopée Européenne (PE 5.0 tome 1, 01/2005 : 20916, paragraphe 2.9.16 ; équipement selon la figure 2-9-16-2). Exactement 100 g de poudre sont versés dans l'entonnoir normalisé dont l'orifice de sortie est obturé. A la libération de cette ouverture, un chronomètre est déclenché puis stoppé à la fin de l'écoulement du produit (entonnoir vide). Les mesures d'écoulement sont exprimées en secondes.

Les valeurs de densité tassée et aérée, et de compressibilité de la poudre de maltitol cristallisé obtenue par le procédé selon l'invention sont déterminées en utilisant l'appareil POWDER TESTER type PTE commercialisé par la société HOSOKAWA, en suivant les spécifications du constructeur.

Cet appareil permet de mesurer, dans des conditions standardisées et reproductibles, l'aptitude à l'écoulement d'une poudre en mesurant notamment la densité aérée vrac et la densité tassée vrac et ensuite de calculer, à partir de ces données, les valeurs de compressibilité par la formule suivante :

$$\text{Compressibilité (\%)} = \frac{(\text{densité tassée} - \text{densité aérée})}{\text{Densité tassée}} \times 100$$

La poudre de maltitol cristallisé obtenue par le procédé conforme à l'invention est donc tout d'abord caractérisée par sa densité tassée et sa densité aérée, cette mesure étant réalisée sur l'appareil POWDER TESTER de type PTE, comme mentionné ci-avant, selon la méthode préconisée dans le mode d'emploi dudit POWDER TESTER (réglage par défaut sur 180 secousses pour la mesure de la densité tassée).

Dans ces conditions, la poudre de maltitol cristallisé obtenue par le procédé conforme à l'invention présente une densité aérée supérieure à 0,85 g/ml, de préférence comprise entre 0,88 et 1,00 g/ml, une densité tassée supérieure à 0,97 g/ml, de préférence comprise entre 0,98 et 1,05 g/ml.

La valeur de compressibilité est également un facteur très important pour caractériser les qualités particulières de la poudre de maltitol cristallisé obtenue par le procédé selon l'invention.

Selon le mode d'emploi de l'appareil PTE d'HOSOKAWA, quand la valeur de compressibilité est d'environ 20 %, la poudre ne présente pas d'écoulement libre et a tendance à former des voûtes dans la trémie. Pour des valeurs particulières de compressibilité de 40 - 50 %, il devient même impossible de décharger le matériel de la trémie une fois que le matériel y a été stocké.

La poudre de maltitol cristallisé obtenue par le procédé conforme à l'invention présente une valeur de compressibilité inférieure à 17 %, de préférence inférieure à 10 %, et encore plus préférentiellement inférieure à 5 %, ce qui correspond à un écoulement tout à fait remarquable, contrairement aux autres poudres de maltitol cristallisé plus riche en fines particules, comme il sera exemplifié ci-après.

Ces paramètres de densité, d'aptitude à l'écoulement et de diamètre moyen rendent la poudre de maltitol cristallisé obtenue par le procédé selon l'invention particulièrement adaptée aux applications auxquelles on la destine.

Une première famille de poudre de maltitol cristallisé obtenue par le procédé conforme à l'invention présente, pour un diamètre moyen arithmétique D4,3 compris entre 200 et 350 μm, une répartition granulométrique en volume avec moins de 20 %, de préférence moins de 15 % de particules de taille inférieure à 200 μm

Une deuxième famille de poudre de maltitol cristallisé obtenue par le procédé conforme à l'invention présente, pour un diamètre moyen arithmétique D4,3 compris entre 450 et 600 μm, une répartition granulométrique en volume avec moins de 10 %, de préférence moins de 5 % de particules de taille inférieure à 200 μm.

La poudre de maltitol cristallisé obtenue par le procédé conforme à l'invention est susceptible d'être obtenue en mettant en oeuvre une technologie de séparation des cristaux de maltitol en fonction de leur taille et de leur poids; surtout un procédé permettant d'extraire la fraction des cristaux de maltitol présentant la taille la plus élevée.

Pour parvenir à ce résultat, la société Demanderesse utilise un séparateur statique, plus communément appelé par l'homme du métier séparateur Zig-Zag (cf. l'enseignement du brevet US 1.861.248).

La séparation dans un sélecteur Zig-Zag est une séparation gravitaire par air. Il s'agit d'un procédé de séparation dans lequel les particules solides sont séparées selon leur comportement lors de leur chute, car dans la zone de séparation, elles sont soumises à la force gravitaire et à la force de traînée du flux d'air. La séparation est en fait basée sur la différence de trajectoires de particules non identiques dans la zone de séparation.

Dans le procédé conforme à l'invention, on choisit un séparateur Zig-Zag à plusieurs étages permettant d'utiliser le même air pour tous les étages, et de répéter la séparation à la fois dans le courant ascendant des particules légères et dans le courant d'air descendant des grosses particules.

Le séparateur est construit en assemblant un nombre de sections ensemble avec un angle fixé afin de créer le canal du Zig-Zag. Le canal a une section rectangulaire. Sa géométrie particulière et la direction du flux d'air induit alors deux courants de particules distincts : un courant de particules légères emportées par le courant d'air ascendant ;

un courant de particules lourdes descendant le long de la paroi la plus basse de chaque section.

A chaque étage, les particules des deux courants sont donc soumises à une nouvelle séparation. Après quoi les particules continuent leurs mouvements dans le courant de particules originales ou sont transportées dans le courant de direction opposée.

La performance du séparateur est déterminée par le comportement des particules à chaque étage d'une part et par l'interaction entre les étages d'autre part.

Dans le procédé conforme à l'invention, le séparateur Zig-Zag utilisé permet de séparer une poudre de maltitol cristallisé en deux fractions (fines et grosses).

Pour ce faire, un jet d'air ascendant (air primaire) est envoyé dans le séparateur Zig-Zag, sa vitesse permettant de caractériser le diamètre de coupure.

Les particules de diamètre supérieur au diamètre de coupure descendent malgré le jet d'air, alors que les autres sont entraînées par l'air ascendant.

Le procédé de préparation de la poudre de maltitol cristallisé selon l'invention consiste à :

a) alimenter un séparateur Zig-Zag présentant un canal composé de plusieurs étages d'inclinaison à 120°, avec une poudre de maltitol cristallisé présentant pour un diamètre moyen arithmétique compris entre 180 et 230 $\mu$m, une répartition granulométrique en volume de :

- plus de 20 % de particules présentant une taille inférieure à 200 $\mu$m,
- plus de 7 % de particules de taille inférieure à 100 $\mu$m,
- plus de 2 % de particules de taille inférieure à 40 $\mu$m,

b) régler le débit d'air primaire de manière à récupérer une fraction de poudre de maltitol cristallisé présentant une répartition granulométrique en volume de :

- moins de 20 %, de préférence moins de 15 %, plus préférentiellement moins de 10 % et plus préférentiellement encore moins de 5 % de particules de taille inférieure à 200 $\mu$m,
- moins de 6 % de particules de taille inférieure à 100 $\mu$m,
- moins de 2 % de particules de taille inférieure à 40 $\mu$m.

Selon un premier mode de réalisation préféré du procédé conforme à l'invention, le procédé de préparation de la poudre de maltitol cristallisé selon l'invention consiste alors à :

a) alimenter un séparateur Zig-Zag présentant un canal composé de 7 étages d'inclinaison 120°, de largeur comprise entre 2 et 3 cm, de longueur comprise entre 4 et 5 cm et d'épaisseur de 4 cm, avec une poudre de maltitol cristallisé présentant pour un diamètre moyen arithmétique compris entre 180 et 230 $\mu$m, une répartition granulométrique en volume de :

- plus de 20 % de particules présentant une taille inférieure à 200 $\mu$m,
- plus de 7 % de particules de taille inférieure à 100 $\mu$m,
- plus de 2 % de particules de taille inférieure à 40 $\mu$m,

à un débit compris entre 400 et 600 g/min,
b) fixer le débit d'air primaire à une valeur comprise entre 2 et 5 m$^3$/h,
c) récupérer la fraction de la poudre présentant pour un diamètre moyen arithmétique compris entre 200 et 350 $\mu$m, une répartition granulométrique en volume de moins 20 %, de préférence moins de 15 % de particules de taille inférieure à 200 $\mu$m.

Selon un deuxième mode de réalisation préféré du procédé conforme à l'invention, le procédé de préparation de la poudre de maltitol cristallisé selon l'invention consiste alors à :

a) alimenter un séparateur Zig-Zag présentant un canal composé de 7 étages d'inclinaison 120°, de largeur comprise entre 2 et 3 cm, de longueur comprise entre 4 et 5 cm et d'épaisseur de 4 cm, avec une poudre de maltitol cristallisé présentant pour un diamètre moyen arithmétique compris entre 180 et 230 $\mu$m, une répartition granulométrique en volume de :

- plus de 20 % de particules présentant une taille inférieure à 200 $\mu$m,
- plus de 7 % de particules de taille inférieure à 100 $\mu$m,

- plus de 2 % de particules de taille inférieure à 40 $\mu$m,

à un débit compris entre 450 et 550 g/min,
b) fixer le débit d'air primaire à une valeur comprise entre 40 et 50 m³/h,
c) récupérer la fraction de la poudre présentant pour un diamètre moyen arithmétique compris entre 450 et 600 $\mu$m, une répartition granulométrique en volume avec moins de 10 %, de préférence moins de 5 % de particules de taille inférieure à 200 $\mu$m.

La poudre de maltitol cristallisé obtenue par le procédé conforme à l'invention peut avantageusement être utilisée dans l'industrie Alimentaire, par exemple dans les domaines du chocolat et des édulcorants de table.

Dans le domaine du chocolat, comme il sera exemplifié ci-après, la quasi absence de fines particules dans la poudre de maltitol cristallisé obtenue par le procédé conforme à l'invention permet alors d'obtenir une meilleure fluidité de la masse après malaxage, un meilleur transport vers le raffinage sans impacter la rhéologie finale du chocolat ainsi fabriqué.

Toutefois, rien n'empêche de l'utiliser à une tout autre fin, comme par exemple dans les domaines :

- du baking (dans la garniture de pâtisseries tels les beignets (« doughnut »), plus particulièrement par son aptitude à l'écoulement, dans les systèmes de dosages et de mélange dans des applications mix de boulangerie/pâtisserie, boulangeries industrielles (facilité de dosage) ou pâtisserie industrielle, et par sa granulométrie, pour la substitution du sucre cristal (dans des fourrages gras par exemple).
- du candisage de confiseries (pâtes de fruits, gommes, gélifiés, sucres cuits),
- des chewing gums (apport de gros cristaux croustillants dans le centre du chewing-gum),
- des fondants,
- des sachets pharmaceutiques,
- des préparations instantanées,
- des supports d'arômes,
- des supports d'édulcorants intenses,
- des céréales et céréales petit-déjeuner (en glaçage), et
- dans les sauces sans sucre ajouté.

Plus particulièrement, la poudre de maltitol obtenue par le procédé conforme à l'invention, présentant un diamètre moyen des particules de maltitol compris entre 450 et 600 $\mu$m, sera choisie pour des applications nécessitant des mélanges à secs pour cuisson, pour la préparation de glaces, pour les boissons en poudre, pour les garnitures de pâtisseries et pour les confiseries.

Elle peut être également avantageusement utilisée pour apporter un caractère croustillant en bouche.

L'invention sera encore mieux comprise à l'aide des exemples qui suivent, lesquels ne se veulent pas limitatifs et font seulement état de certains modes de réalisation et de certaines propriétés avantageuses de la poudre de maltitol cristallisé obtenue par le procédé selon l'invention.

**Exemple 1**

On introduit du MALTISORB® P200 dans la trémie d'alimentation d'un séparateur MULTIPLEX ZIGZAG CLASSIFIER 1-40 de la société HOSOKAWA, muni de 7 étages d'inclinaison 120°, présentant à chaque étage une largeur de 2 cm, une longueur de 4 cm et une épaisseur de 4 cm.

Deux conduites de séparation sont menées afin d'obtenir deux poudres de maltitol cristallisé définées présentant respectivement :

- un diamètre moyen des particules de maltitol compris entre 200 et 350 $\mu$m (produit « A »),
- un diamètre moyen des particules de maltitol compris entre 450 et 600 $\mu$m (produit « B »).

Pour cela, on adapte surtout le débit d'alimentation d'air primaire.

La vitesse de l'air ascendant définit en effet le diamètre de coupure du mélange initial.

C'est ainsi qu'en partant de la même poudre de maltitol cristallisé, en l'occurrence ici le MALTISORB® P200, la mise en oeuvre d'un débit d'air primaire à une valeur de l'ordre de :

- 3,4 m³/h, permet d'obtenir une poudre de maltitol cristallisé « définée » (présentant de l'ordre de 6 % de particules de taille inférieure à 100 $\mu$m et de l'ordre de 2 % de particules inférieures à 40 $\mu$m
- 45 m³/h (soit un débit treize fois plus élevé), permet de baisser encore plus la teneur en fines particules de 100 et

40 $\mu$m (respectivement à 2,1 % et 0,7 %), mais surtout de baisser de façon remarquable la teneur en particules de moins de 200 $\mu$m (de 19,6 % à 5,4 %).

Les conditions de mise en oeuvre sont présentées dans le tableau 1 suivant.

Tableau 1

| Poudre de maltitol obtenue par le procédé selon l'invention | Débit poudre (g/min) | Débit Air primaire (m³/h) | Poids Fractions (g) | | | Alimentation (μm) | | | | Fraction définie (μm) | | | |
|---|---|---|---|---|---|---|---|---|---|---|---|---|---|
| | | | Poids total | Poids fines | Poids grosses | Diamètre arithmétique moyen (μm) D(4,3) | % en volume >40 | % en volume <100 | % en volume <200 | Diamètre arithmétique moyen (μm) D(4,3) | % en volume >40 | % en volume <100 | % en volume <200 |
| Produit « A » | 500 | 3,4 | 7450 | 3138 | 4312 | 219 | 4,8 | 23,2 | 53,7 | 280 | 2,0 | 6,0 | 19,6 |
| Produit « B » | 492 | 45 | 7378 | 450 | 6928 | 219 | 4,8 | 23,2 | 53,7 | 519 | 0,7 | 2,1 | 5,4 |

Les mesures d'écoulement, de densités aérées et tassées, ainsi que de compressibilité, ont été réalisées à l'aide des méthodes décrites ci-dessus.

Le produit « A » présente une valeur d'écoulement de 5 secondes, et présente par ailleurs :

- une densité aérée de 0,885 g/ml,
- une densité tassée de 1,025 g/ml, et
- une compressibilité de 13,655 %.

Le produit « B » présente quant à lui une valeur d'écoulement de 7 secondes, et présente par ailleurs :

- une densité aérée de 0,96 g/ml,
- une densité tassée de 0,98 g/ml, et
- une compressibilité de 2,04 %.

**Exemple 2**

La répartition granulométrique du maltitol dans le chocolat est mesurée en comparant des chocolats fabriqués avec deux poudres de maltitol cristallisé obtenues par le procédé conformes à l'invention (Produit « A » et Produit « B » de l'exemple 1) par rapport à ceux préparés d'une part avec du saccharose (sucre cristal n°1-600 de TEREOS), et d'autre part avec une poudre de maltitol cristallisé de l'art antérieur recommandée pour cette application, présentant un diamètre moyen arithmétique D4,3 compris entre 180 et 230 $\mu$m, une répartition granulométrique en volume de :

- plus de 20 % de particules présentant une taille inférieure à 200 $\mu$m,
- plus de 7 % de particules de taille inférieure à 100 $\mu$m,
- plus de 2 % de particules de taille inférieure à 40 $\mu$m, tel le MALTISORB® P 200 commercialisé par la société Demanderesse.

La recette utilisée pour la fabrication des chocolats est présentée dans le tableau 2 suivant :

Tableau 2

| Recette du chocolat | Composition en % | % matière grasse |
|---|---|---|
| Poudre de maltitol cristallisé obtenue par le procédé selon l'invention, ou poudre de maltitol cristallisé MALTISORB® P 200, ou Sucre Cristal 600 | 43,5 | 0,00 |
| Liqueur de cacao | 19,5 | 10,53 |
| Poudre de cacao | 19,5 | 1,95 |
| Beurre de cacao | 17 | 17,000 |
| Lécithine | 0,5 | 0,50 |
| **Total** | **100** | **29,98** |

La formule est la même pour tous les essais, seules les matières sucrantes diffèrent.

Le malaxage est effectué dans un mélangeur planétaire ou dans un pétrin, le raffinage est effectué sur broyeuse tricylindre.

La pression entre les rouleaux est la suivante :

- 1er passage : 20 - 30 bars
- 2ème passage : 35 - 45 bars
- 3ème passage : 55 - 60 bars.

La composition au malaxage /raffinage est donnée dans le tableau 3 suivant :

Tableau 3

|  | Composition en % | % matière grasse |
|---|---|---|
| Poudre de maltitol cristallisé obtenue par le procédé selon l'invention, ou poudre de maltitol cristallisé MALTISORB® P 200, ou Sucre Cristal 600 | 48,1 | 0 |
| Liqueur de cacao | 21,55 | 11,64 |
| Poudre de cacao | 21,55 | 2,15 |
| Beurre de cacao | 8,8 | 8,84 |
| **Total** | **100** | **22,63** |

Le conchage est réalisé à 60°C pendant 14 h.

La composition au conchage est donnée dans le tableau 4 suivant :

Tableau 4

|  | Composition (%) |
|---|---|
| Poudre issue du raffinage | 90,5 |
| Beurre de cacao | 9,0 |
| Lécithine | 0,5 |
| **Total** | **100** |

Les poudres de maltitol cristallisé ainsi que le sucre Cristal 600 ont été caractérisés par granulométrie laser avant leur utilisation dans le chocolat. Les mesures de granulométrie ont été réalisées au moyen d'un granulomètre laser BECKMANN COULTER LS 230.

Tableau 5

|  | Diamètre moyen arithmétique (μm) | % de particules inférieures à : | | |
|---|---|---|---|---|
|  |  | 40 μm | 100 μm | 200 μm |
| MALTISORB® P200 | 219 | 4,8 | 23,2 | 53,7 |
| Produit « A » | 280 | 2,0 | 6,0 | 19,6 |
| Produit « B » | 519 | 0,7 | 2,1 | 5,4 |
| Saccharose | 660 | 0,3 | 0,9 | 2,4 |

Ces résultats montrent bien une répartition granulométrique très différente entre le MALTISORB® P200 et les poudres de maltitol obtenues par le procédé conforme à l'invention. La répartition granulométrique des poudres de maltitol obtenues par le procédé conforme à l'invention, et plus particulièrement pour le produit « B », est plus proche du saccharose que la répartition granulométrique du MALTISORB® P200.

Les mesures de granulométrie ont été également réalisées sur les chocolats (produits finis) afin de déterminer si, après raffinage et conchage, les différences entre le MALTISORB® P200, la poudre de maltitol cristallisé obtenue par le procédé de l'invention et le saccharose persistent.

La détermination de la granulométrie des particules de maltitol dans le chocolat est effectuée par toute méthode connue par ailleurs de l'homme du métier et peut consister, par exemple, à disperser du chocolat cassé en morceaux dans du propanol 2, puis, dans le granulomètre à diffraction LASER type LS 230 de la société BECKMAN-COULTER, sélectionner la nature du fluide comme étant le propanol 2 et utiliser le modèle optique adapté en suivant les spécifications du constructeur.

Le tableau 6 suivant résume les résultats obtenus.

Tableau 6

| | Diamètre moyen arithmétique (µm) | % de particules inférieures à: | | |
|---|---|---|---|---|
| | | 40 µm | 100 µm | 200 µm |
| Chocolats avec MALTISORB® P200 | 13,4 | 96,4 | 99,3 | 100,0 |
| Chocolats avec le Produit « A » | 11,2 | 96,5 | 99,5 | 100 |
| Chocolats avec le Produit « B » | 10,4 | 96,4 | 100,0 | 100,0 |
| Chocolats avec le saccharose | 15,3 | 90,5 | 99,6 | 100,0 |

L'utilisation des poudres de maltitol cristallisé obtenues par le procédé conforme à l'invention dans le chocolat donne après conchage une répartition de taille de particules assez proche de celle obtenue avec le MALTISORB® P200 et le saccharose.

Ce résultat démontre que le raffinage des poudres de maltitol cristallisé obtenues par le procédé conforme à l'invention est aussi efficace que celui du MALTISORB® P200 ou du saccharose.

On étudie ensuite le comportement rhéologique de la masse de chocolat, après malaxage et avant raffinage, en fonction de la répartition granulométrie des poudres de maltitol cristallisé testées.

Les masses de chocolat ne s'écoulant pas à ce stade du procédé (la pâte est bien trop épaisse), la mesure a été effectuée par pénétrométrie.

On mesure la force de pénétration INSTRON des masses de chocolat fabriquées avec les deux poudres de maltitol cristallisé obtenues par le procédé conforme à l'invention, et en témoins, avec du MALTISORB® P200 ou du saccharose.

Les mesures sont effectuées sur un pénétromètre INSTROM de type 4502, avec une cellule de 100 N, à l'aide d'un poinçon boule de 10 mm de diamètre. Le déplacement est imposé à 20 mm, et la vitesse de traverse est de 10 mm/min.

Les masses de chocolat ont été stockées à une température de 50°C, et les mesures effectuées à cette température.

Les forces ont été mesurées à 10 mm de pénétration.

Le tableau 7 suivant présente les résultats obtenus, qui montrent que la masse de chocolat préparée à partir des poudres de maltitol cristallisé obtenue par le procédé selon l'invention présente une résistance à la pénétration significativement plus faible que celle préparée à partir du MALTISORB P200, ce qui laisse augurer d'un bien meilleur écoulement du malaxeur vers le raffinage.

Tableau 7

| | Force (N) |
|---|---|
| Poudre « A » | 0,8 |
| Poudre « B » | 0,5 |
| MALTISORB® P200 | 2,1 |
| Saccharose | 0,4 |

La masse de chocolat préparée à partir des poudres de maltitol cristallisé obtenue par le procédé de l'invention est en effet plus mobile, ce qui confirme bien l'impact de l'absence de fines particules sur la viscosité de la masse de chocolat ainsi préparée. Les forces de pénétration des pâtes de chocolat préparées à partir des poudres de maltitol cristallisé de l'invention se rapprochent de celle de la pâte de chocolat préparée avec du saccharose.

En conclusion, ces résultats montrent que la rhéologie du chocolat, après malaxage, est influencée par la répartition granulométrique de la poudre de maltitol cristallisé.

Celles obtenues par le procédé conforme à l'invention permettent ainsi d'obtenir des pâtes plus molles que celle à base de MALTISORB® P200, pâtes se rapprochant de la pâte à base de saccharose.

Ces pâtes seront ainsi plus faciles à extraire du malaxeur pour leur transfert vers les étapes ultérieures du process (raffinage, conchage).

Ce résultat augure également de la possibilité de l'usage de moins de matière grasse au moment du malaxage (ce qui rend possible une texture identique au produit préparé à partir du MALTISORB® P200), afin d'ajouter davantage de matière grasse libre au moment du conchage, et ainsi obtenir une fluidité plus importante à la coulée.

Cet avantage technologique permet une meilleure mise en oeuvre des ingrédients au moment du process de fabrication du chocolat.

Les poudres de maltitol cristallisé obtenues par le procédé selon l'invention facilitent les premières étapes du process de fabrication du chocolat, sans modifier la texture finale obtenue.

Ainsi, le Tableau 8 montre les caractéristiques rhéologiques mesurées sur les chocolats en final (après conchage).

Tableau 8

|  | Viscosité Casson (Pa.s) | Seuil d'écoulement (Pa) |
|---|---|---|
| Chocolat avec MALTISORB® P200 | 3,1 | 51 |
| Chocolat avec produit « A » | 3,3 | 48 |
| Chocolat avec produit « B » | 3,1 | 47 |
| Saccharose | 3,7 | 52 |

Les différences observées ici ne sont pas significatives, démontrant une qualité préservée des chocolats fabriqués, et ce quelle que soit la matière sucrante utilisée.

**Exemple 3**

Pour la fabrication des édulcorants de table on introduit 960 g de poudre de maltitol cristallisé de l'exemple 1 (Produit « B ») dans la cuve d'un granulateur-sécheur à lit d'air fluidisé de laboratoire type STREA-1 de Aeromatic-Fielder AG.

On procède de la même façon avec le MALTISORB® P200, pris ici en témoin.

On prépare une solution de 40 g de NUTRIOSE® FB06, 2,2 g de sucralose avec 100 g d'eau.

L'air de fluidification du Produit « B » comme du MALTISORB® P200 est chauffé à 60°C.

On pulvérise la solution préparée à un débit de 300 ml/h par une buse positionnée en haut de cuve.

Après la pulvérisation, on sèche pendant 30 mn à 60°C. Les résultats obtenus figurent dans le tableau 9 suivant.

Tableau 9

| Poudre de maltitol testée | Produit « B » | MALTISORB® P 200 |
|---|---|---|
| Densité aérée (q/ml) | 0,8 | 0,61 |
| Densité tassée (g/ml) | 0,83 | 0,65 |
| Compressibilité (%) | 3,6 | 6,1 |
| Apparence | cristaux | Granules |

On constate que les valeurs des densités des préparations restent avantageusement élevées avec la poudre de maltitol cristallisé obtenue par le procédé conforme à l'invention (> 0.75 g/ml pour la densité aérée) contre -0.60 g/ml pour le MALTISORB® P200.

Des différences sont également observées au niveau compressibilité : moins de compaction pour les préparations à base de poudre de maltitol cristallisé obtenue par le procédé conforme à l'invention, ce qui leur confère une bien meilleure fluidité et facilité de manipulation.

Quant à la conduite de fabrication, on constate que lors du procédé de granulation de la poudre de maltitol cristallisé obtenue par le procédé conforme à l'invention avec le NUTRIOSE® FB06, la fluidisation est favorisée dans le granulateur à lit d'air fluidisé car le mélange est plus homogène qu'avec le MALTISORB® P200.

De plus, on constate une bien meilleure productivité car il y a moins de perte de matière par collage des fines particules sur les filtres et les parois du matériel.

Enfin, l'aspect des édulcorants de table fabriqués à partir du MALTISORB® P200 s'apparente plus à des granulés, alors que celui des édulcorants de table préparés à partir de la poudre de maltitol obtenue par le procédé conforme à l'invention est plutôt celui d'un monocristal (aspect tout à fait similaire au saccharose cristallisé de grosse taille).

La perception visuelle des édulcorants de table préparés à partir de la poudre de maltitol obtenue par le procédé conforme à l'invention est donc bien meilleure, et permet d'atteindre l'un des objectifs clefs de la perception des édulcorants intenses par les consommateurs : celui de faire oublier à l'utilisateur final qu'il utilise des édulcorants intenses.

**Revendications**

1. Procédé de préparation d'une poudre de maltitol cristallisé, **caractérisé en ce qu'**il consiste à :

a) alimenter un séparateur Zig-Zag présentant un canal composé de plusieurs étages d'inclinaison à 120°, avec une poudre de maltitol cristallisé présentant pour un diamètre moyen arithmétique compris entre 180 et 230 $\mu$m, une répartition granulométrique en volume de :

- plus de 20 % de particules présentant une taille inférieure à 200 $\mu$m,
- plus de 7 % de particules de taille inférieure à 100 $\mu$m,
- plus de 2 % de particules de taille inférieure à 40 $\mu$m,

b) régler le débit d'air primaire de manière à récupérer une fraction de poudre de maltitol cristallisé présentant une répartition granulométrique en volume de :

- moins de 20 %, de préférence moins de 15 %, plus préférentiellement moins de 10 % et plus préférentiellement encore moins de 5 % de particules de taille inférieure à 200 $\mu$m,
- moins de 6 % de particules de taille inférieure à 100 $\mu$m,
- moins de 2 % de particules de taille inférieure à 40 $\mu$m.

2. Procédé selon la revendication 1, **caractérisé en ce qu'**il consiste à :

a) alimenter un séparateur Zig-Zag présentant un canal composé de 7 étages d'inclinaison 120°, de largeur comprise entre 2 et 3 cm, de longueur comprise entre 4 et 5 cm et d'épaisseur de 4 cm, avec une poudre de maltitol cristallisé présentant pour un diamètre moyen arithmétique compris entre 180 et 230 $\mu$m, une répartition granulométrique en volume de :

- plus de 20 % de particules présentant une taille inférieure à 200 $\mu$m,
- plus de 7 % de particules de taille inférieure à 100 $\mu$m,
- plus de 2 % de particules de taille inférieure à 40 $\mu$m,

à un débit compris entre 400 et 600 g/min,
b) fixer le débit d'air primaire à une valeur comprise entre 2 et 5 m$^3$/h,
c) récupérer la fraction de la poudre présentant pour un diamètre moyen arithmétique compris entre 200 et 350 $\mu$m, une répartition granulométrique en volume de moins 20 %, de préférence moins de 15 % de particules de taille inférieure à 200 $\mu$m.

3. Procédé selon la revendication 1, **caractérisé en ce qu'**il consiste à :

a) alimenter un séparateur Zig-Zag présentant un canal composé de 7 étages d'inclinaison 120°, de largeur comprise entre 2 et 3 cm, de longueur comprise entre 4 et 5 cm et d'épaisseur de 4 cm, avec une poudre de maltitol cristallisé présentant pour un diamètre moyen arithmétique compris entre 180 et 230 $\mu$m, une répartition granulométrique en volume de :

- plus de 20 % de particules présentant une taille inférieure à 200 $\mu$m,
- plus de 7 % de particules de taille inférieure à 100 $\mu$m,
- plus de 2 % de particules de taille inférieure à 40 $\mu$m,

à un débit compris entre 450 et 550 g/min,
b) fixer le débit d'air primaire à une valeur comprise entre 40 et 50 m$^3$/h,
c) récupérer la fraction de la poudre présentant pour un diamètre moyen arithmétique compris entre 450 et 600 $\mu$m, une répartition granulométrique en volume avec moins de 10 %, de préférence moins de 5 % de particules de taille inférieure à 200 $\mu$m.

**Patentansprüche**

1. Verfahren zur Herstellung von kristallisiertem Maltitol-Pulver, **dadurch gekennzeichnet, dass** es umfasst:

a) Zuführen eines kristallisierten Maltitol-Pulvers, welches bei einem arithmetischen Mitteldurchmesser von zwischen 180 und 230 μm eine Partikelgrößenverteilung in Volumen aufweist von:

- mehr als 20% der Partikel mit einer Größe von weniger als 200 μm,
- mehr als 7% der Partikel mit einer Größe von weniger als 100 μm,
- mehr als 2% der Partikel mit einer Größe von weniger als 40 μm, an einen Zick-Zack-Abscheider, der einen Kanal bestehend aus mehreren Stufen mit einer Neigung von 120° aufweist,

b) Einstellen der Primärluftzufuhr in einer Weise, um eine Fraktion des kristallisierten Maltitol-Pulvers wiederzugewinnen, welche eine Partikelgrößenverteilung in Volumen aufweist von:

- weniger als 20%, vorzugsweise weniger als 15%, mehr bevorzugt weniger als 10% und noch mehr bevorzugt weniger als 5% der Partikel mit einer Größe von weniger als 200 μm,
- weniger als 6% der Partikel mit einer Größe von weniger als 100 μm,
- weniger als 2% der Partikel mit einer Größe von weniger als 40 μm.

2. Verfahren nach Anspruch 1, **dadurch gekennzeichnet, dass** es umfasst:

a) Zuführen eines kristallinen Maltitol-Pulvers, welches bei einem arithmetischen Mitteldurchmesser von zwi-schen 180 und 230 μm eine Partikelgrößenverteilung in Volumen aufweist von:

- mehr als 20% der Partikel mit einer Größe von weniger als 200 μm,
- mehr als 7% der Partikel mit einer Größe von weniger als 100 μm,
- mehr als 2% der Partikel mit einer Größe von weniger als 40 μm, an einen Zick-Zack-Abscheider, welcher einen Kanal bestehend aus 7 Stufen mit einer Neigung von 120°, eine Breite zwischen 2 und 3 cm, eine Länge zwischen 4 und 5 cm und eine Dicke von 4 cm aufweist,

bei einem Durchsatz zwischen 400 und 600 g/min
b) Einstellen der Primärluftzufuhr auf einen Wert zwischen 2 und 5 m$^3$/Stunde,
c) Wiedergewinnen der Fraktion des Pulvers, welches bei einem arithmetischen Mitteldurchmesser von zwi-schen 200 und 350 μm eine Partikelgrößenverteilung in Volumen von weniger als 20%, vorzugsweise weniger als 15% der Partikel mit einer Größe von weniger als 200 μm aufweist.

3. Verfahren nach Anspruch 1, **dadurch gekennzeichnet, dass** es umfasst:

a) Zuführen eines kristallinen Maltitol-Pulvers, welches bei einem arithmetischen Mitteldurchmesser von zwi-schen 180 und 230 μm eine Partikelgrößenverteilung in Volumen aufweist von:

- mehr als 20% der Partikel mit einer Größe von weniger als 200 μm,
- mehr als 7% der Partikel mit einer Größe von weniger als 100 μm,
- mehr als 2% der Partikel mit einer Größe von weniger als 40 μm,

an einen Zick-Zack-Abscheider, welcher einen Kanal bestehend aus 7 Stufen mit einer Neigung von 120°, eine Breite zwischen 2 und 3 cm, eine Länge zwischen 4 und 5 cm und eine Dicke von 4 cm aufweist, bei einem Durchsatz zwischen 450 und 550 g/min
b) Einstellen der Primärluftzufuhr auf einen Wert zwischen 40 und 50 m$^3$/Stunde,
c) Wiedergewinnen der Fraktion des Pulvers, welches bei einem arithmetischen Mitteldurchmesser von zwi-schen 450 und 600 μm eine Partikelgrößenverteilung in Volumen von weniger als 10%, vorzugsweise weniger als 5% der Partikel mit einer Größe von weniger als 200 μm aufweist.

**Claims**

1. A process of preparing of the crystallized maltitol powder according to any one of claims 1 to 3, **characterized in that** it comprises:

a) feeding a zig-zag separator having a channel composed of several stages with a 120° slope with a crystallized maltitol powder having, for an arithmetic mean diameter comprised between 180 and 230 μm, a particle-size

distribution by volume of:

- more than 20% particles of less than 200 $\mu$m in size,
- more than 7% particles of less than 100 $\mu$m in size,
- more than 2% particles of less than 40 $\mu$m in size,

b) controlling the flow rate of primary air so as to recover a fraction of crystallized maltitol powder having a particle-size distribution by volume of:

- less than 20%, preferably less than 15%, more preferably less than 10% and even more preferably less than 5% particles of less than 200 $\mu$m in size,
- less than 6% particles of less than 100 $\mu$m in size,
- less than 2% particles of less than 40 $\mu$m in size.

2. The process according to claim 1, **characterized in that** it comprises:

a) feeding a zig-zag separator having a channel composed of 7 stages with a 120° slope, with a width comprised between 2 and 3 cm, a length comprised between 4 and 5 cm and a thickness of 4 cm, with a crystallized maltitol powder having, for an arithmetic mean diameter comprised between 180 and 230 $\mu$m, a particle-size distribution by volume of:

- more than 20% particles of less than 200 $\mu$m in size,
- more than 7% particles of less than 100 $\mu$m in size,
- more than 2% particles of less than 40 $\mu$m in size,

at a flow rate comprised between 400 and 600 g/min,
b) fixing the flow rate of primary air at a value comprised between 2 and 5 m$^3$/h,
c) recovering the fraction of the powder having, for an arithmetic mean diameter comprised between 200 and 350 $\mu$m, a particle-size distribution by volume of less than 20%, preferably less than 15% particles of less than 200 $\mu$m in size.

3. The process according to claim 1, **characterized in that** it comprises:

a) feeding a zig-zag separator having a channel composed of 7 stages with a 120° slope, with a width comprised between 2 and 3 cm, a length comprised between 4 and 5 cm and a thickness of 4 cm, with a crystallized maltitol powder having, for an arithmetic mean diameter comprised between 180 and 230 $\mu$m, a particle-size distribution by volume of:

- more than 20% particles of less than 200 $\mu$m in size,
- more than 7% particles of less than 100 $\mu$m in size,
- more than 2% particles of less than 40 $\mu$m in size,

at a flow rate comprised between 450 and 550 g/min,
b) fixing the flow rate of primary air at a value comprised between 40 and 50 m$^3$/h,
c) recovering the fraction of the powder having, for an arithmetic mean diameter comprised between 450 and 600 $\mu$m, a particle-size distribution by volume with less than 10%, preferably less than 5% particles of less than 200 $\mu$m in size.

**EP 2 249 870 B1**

**RÉFÉRENCES CITÉES DANS LA DESCRIPTION**

**Documents brevets cités dans la description**

- EP 512910 A
- EP 189704 A
- EP 1245582 A
- EP 1006128 A
- US 1861248 A